# NEW EUROPEAN PATENT SPECIFICATION

(11) **EP 2 056 787 B2**
(45) Date of publication and mention of the opposition decision: **03.05.2017**
(45) Mention of the grant of the patent: 22.01.2014
(21) Application number: 07841570.0
(22) Date of filing: 30.08.2007
(51) Int. Cl.: C11D 1/72

(54) **DENTURE CARE COMPOSITION**
ZAHNPFLEGEZUSAMMENSETZUNG
COMPOSITION DE SOINS DE PROTHÈSES DENTAIRES

(30) Priority: 01.09.2006 US 842202 P
(43) Date of publication of application: 13.05.2009
(73) Proprietor: GlaxoSmithKline LLC, Wilmington DE 19808 (US)
(72) Inventor: DULLEA, Charles, V., Parsippany, NJ 07054 (US); LECH, Stanley, J., Parsippany, NJ 07054 (US); DENG, Fang, Scottsdale, AZ 85254 (US)
(74) Representative: Reeves, Julie Frances
(86) International application number: PCT/US2007/077167
(87) International publication number: WO 2008/028003

(56) References cited:
- EP-A2- 0 522 668
- WO-A1-2005/095467
- WO-A2-2008/016869
- GB-A- 1 507 356
- US-A- 4 315 779
- US-A- 4 511 486
- US-A- 5 330 746
- US-A1- 2006 024 246
- US-B1- 6 197 331

## Description

### FIELD OF THE INVENTION

This invention relates to a new denture care composition and method of use. In particular, this invention relates to a film-forming denture care composition that kills odor causing bacteria, maintains prolonged fresh breath for two or more hours and is an effective guard against denture odor.

### BACKGROUND OF THE INVENTION

Oral or denture malodor, as well as bad breath, is a common complaint among denture patients. Due to the artificial nature of the denture appliance, many edentulous patients express concern that they may produce a distinct malodor that is specifically attributable to the presence of their denture. In general, the potential sources of malodour remain the same for all subjects predominated by the production of volatile sulfur compounds (VSC's) by anaerobes, the putrefaction of saliva and food debris, and dietary-related malodor. However, compared to people with natural teeth, the presence of dentures in edentulous patients creates another environment with different surface substrates and its own microflora that build up on the denture during the day causing denture odor. Many denture patients become more concerned about denture odor or bad breath because they try to avoid giving away the fact that they wear dentures. Some of them avoid getting close to people because they worry about their breath. Therefore, the consequences of oral/ denture malodor can be more than cosmetic; they can bring individual denture wearer personal discomfort and social embarrassment.

While denture patients frequently complain of bad breath, it is difficult to propose a treatment regimen beyond frequent cleaning and soaking the dentures, the supporting tissues, and the posterior dorsum of the tongue where odor causing bacteria have been found in dentate patients. Requiring multiple applications per day in order to maintain a feeling of fresh breath can be an all day chore for denture wearers. There are very few commercially available products that can be used by denture patients to treat their bad breath while providing prolonged fresh breath and denture odor control benefits in the oral cavity for more than two hours.

In view of these limitations, finding a denture product that is applied easily and does not require extended cleansing times while maintaining prolonged fresh breath, is an ongoing task. Acceptable agents should be non-toxic and non-staining. In addition, the product should be easy to dispense and apply to the dentures. Maintaining prolonged fresh breath effects without having to clean the dentures multiple times per day is also extremely desirable. The inventors have conceived a denture product that can provide denture wearers the long lasting benefits of oral and dental malodour control and fresh breath for more than a two hour period of time.

WO2005/095467 discloses a composition which is used as intermediate solution for dentures comprising a ternary polymer which has an acrylic acid lower alkyl, a methacrylic-acid lower alkyl, and a methacrylic-acid trimethylchloride ammonium ethyl as a structural component, for instance Eudragit RS100 and comprises water as solvent.

EP0522668 discloses a polymer suitable for use as an active constituent of oral hygiene compositions effective for preventing the adherent deposition of cariogenic bacteria on teeth which polymer comprises defined repeating units comprising hydrocarbyl groups with pendant carboxyl and pendant polyalkylene oxide groups in defined ratio.

GB1507356 discloses a denture cleanser comprising a polyvinylpyrrolidone and a water solvent vehicle.

US6197331 discloses a composition for the care of teeth and dentures comprising a sustained release polymer selected from Eudragit L-100 and a solvent vehicle selected from alcohol and water.

US5330746 discloses an oral composition for plaque prevention or tooth hypersensitivity comprising either an antibacterial agent or a hypersensitivity agent embedded in a sustained release carrier such as an acrylic polymer selected from the group of Eudragit and more preferably Eudragit L-100. The solvent vehicle is polyethylene glycol.

US2006/0024246 discloses oral care compositions comprising a copolymer of methacrylic acid and methyl methacrylate and a carrier comprising a hydrophilic organic polymer such as polyethylene glycol.

### SUMMARY OF THE INVENTION

In one aspect, this invention relates to a liquid denture care composition free of an antimicrobial agent and ethanol, comprising one or more sustained release polymers selected from a copolymer or terpolymer of methacrylic acid and methyl methacrylate; a polyvinyl acetate polymer; a copolymer of ethyl acrylate and methyl methacrylate; a copolymer of polyvinyl alcohol and polyethylene glycol; a copolymer of methacrylic acid and ethyl acrylate; a copolymer of vinylpyrrolidone and vinyl acetate; and a polyvinylpyrrolidone homopolymer, in a solvent vehicle which is propylene glycol, present in the amount of between 50 and 90 weight percent of the total composition.

In another aspect, the disclosure relates to a method for maintaining prolonged fresh breath in a denture wearer comprising applying a liquid denture care composition, free of an antimicrobial agent and ethanol, to the denture outside the mouth, said composition comprising one or more sustained release polymers selected from a copolymer or terpolymer of methacrylic acid and methyl methacrylate; a polyvinyl acetate polymer; a copolymer of ethyl acrylate and methyl methacrylate; a copolymer of polyvinyl alcohol and polyethylene glycol; a copolymer of methacrylic acid and ethyl acrylate; a copolymer of vinylpyrrolidone and vinyl acetate; and a polyvinylpyrrolidone homopolymer, in a solvent vehicle which is propylene glycol, present in the amount of between 50 and 90 weight percent of the total composition, and inserting the denture into the denture wearer's mouth.

In still another aspect, the disclosure relates to a method for the treatment and/or prophylaxis of denture odor and mouth odor in a denture wearer comprising applying a liquid denture care composition free of an antimicrobial agent and ethanol to the denture outside the mouth, said composition comprising one or more sustained release polymers selected from a copolymer or terpolymer of methacrylic acid and methyl methacrylate; a polyvinyl acetate polymer; a copolymer of ethyl acrylate and methyl methacrylate; a copolymer of polyvinyl alcohol and polyethylene glycol; a copolymer of methacrylic acid and ethyl acrylate; a copolymer of vinylpyrrolidone and vinyl acetate; and a polyvinylpyrrolidone homopolymer, in a solvent vehicle which is propylene glycol present in the amount of between 50 and 90 weight percent of the total formulation., and inserting the denture into the denture wearer's mouth.

In yet another aspect, this invention relates to a kit for delivering a liquid denture care composition free of an antimicrobial agent and ethanol having a dispenser containing the denture care composition comprising one or more sustained release polymers selected from a copolymer or terpolymer of methacrylic acid and methyl methacrylate; a polyvinyl acetate polymer; a copolymer of ethyl acrylate and methyl methacrylate; a copolymer of polyvinyl alcohol and polyethylene glycol; a copolymer of methacrylic acid and ethyl acrylate; a copolymer of vinylpyrrolidone and vinyl acetate; and a polyvinylpyrrolidone homopolymer, in a solvent vehicle which is propylene glycol, present in the amount of between 50 and 90 weight percent of the total composition.

### DETAILED DESCRIPTION OF THE INVENTION

The terms "denture" or "partial denture" are used herein interchangeably to refer to artificial teeth, removable orthodontic bridges and denture plates, both upper and lower types orthodontic retainers and appliances, protective mouthguards, nightguards to prevent bruxism and/or Temporomandibular joint (TMJ) disorder, and the like.

The term "denture freshener" is used herein to refer to a formulation for application outside the mouth to provide freshness to dentures or partial dentures.

The term "efficacious amount" is used herein to refer to an amount or concentration of the composition suitable to be effective in providing prolonged fresh breath.

The term "prolonged fresh breath" is used herein to refer to a combination of mouth-feel, denture odor control and mouth odor control sustainable for two or more hours.

The term "free of an antimicrobial agent" is used herein to refer to the fact that the instant composition does not include recognized oral antimicrobial agents, such as, cetyl pyridinium chloride and benzylalkonium chloride. However, one of skill in the art would understand that the instant composition can be used for drug delivery of various therapeutic agents, such as antimicrobial agents which are not included among the recognized oral antimicrobial agents (see list below).

The term "sustained release" is used herein to refer to the continuous release of a substance at efficacious levels for a prolonged period of time, such as two or more hours.

The denture care composition of this invention provides a new product concept for denture wearers. The composition is in the form of a spray-on or brush-on, clear or transparent liquid varnish or gel. The denture wearer applies an efficacious amount of the composition to a clean denture surface. Once applied to the clean denture surface, the product forms a film barrier on the denture surface. Suitably, the coated denture can be inserted into oral cavity without being completely dried. Upon contact with saliva, over time, the coating barrier is believed to slowly release ingredients to provide prolonged fresh breath. Further, the denture care product claimed herein may act as an effective guard or barrier against odor-causing organisms from adhering to the denture.

The composition may be flavored or unflavored. Suitably the denture composition contains a flavorant leaving the denture wearers mouth feeling clean and fresh for two or more hours without interfering with the taste of food. Suitably the flavorant comprises one or more essential oils selected from eucalyptus, cornmint oil, methyl salicylate, lemon, peppermint, tea tree common, tea tree, thyme, spearmint, menthol, and combinations thereof. The major benefit that this formulation provides is improved denture odor control and fresh mouth-taste which lasts two or more hours. Therefore, this invention describes a denture care composition which is designed to kill bacteria, impart a fresh taste and, freshen breath for two or more hours. Without being bound to any particular theory of the invention, it is believed that one or more of the components of the composition are contributing certain antimicrobial properties, thus allowing prolonged fresh breath and taste. For example, it is believed that the essential oils making up the flavorant and/or the solvent vehicle may impart antimicrobial properties to the composition. In any event, the present inventors find it is unnecessary to include recognized oral antimicrobial agents, such as, cetyl pyridinium chloride and benzylalkonium chloride, in order to achieve an antimicrobial effect.

In the invention, the solvent vehicle is propylene glycol in the amount of between 50 and 90 weight percent of the total formulation. In one embodiment of the invention, the solvent vehicle is propylene glycol in the amount of between 65 and 90 weight percent of the total formulation. In one embodiment of the invention, the solvent vehicle is propylene glycol in the amount of between 80 and 87 weight percent of the total formulation.

The composition also contains one or more hydrophobic film-forming polymers which form a homogeneous solution with propylene glycol and which are suitably capable of sustained release. Such sustained release polymers include, but are limited to, copolymers or terpolymers based on an acrylic or methylacrylic acid and an acrylate or a methylacrylate available as Eudragit® polymers; polyvinyl acetate polymers; polyvinyl acetate phthalate (PVAP); a copolymer of ethyl acrylate and methyl methacrylate; a copolymer of polyvinyl alcohol and polyethylene glycol; a copolymer of methacrylic acid and ethyl
acrylate; a copolymer of vinylpyrrolidone and vinyl acetate; and polyvinylpyrrolidone homopolymers.

In one embodiment of the invention, the film-forming polymer is a copolymer or terpolymer based on an acrylic or methylacrylic acid and an acrylate or a methylacrylate polymer in the amount of between 2.0 and 15.0 weight percent of the total formulation. In one embodiment of the invention, the film-forming polymer is a a copolymer or terpolymer based on an acrylic or methylacrylic acid and an acrylate or a methylacrylate polymer in the amount of between 5.0 and 12.0 weight percent of the total formulation.

In addition to the film-forming polymer, one or more excipients suitably may be added to the instant composition. Such excipients include, but are not limited to, antimicrobial enhancing agents, plasticizers, flavorants, control release agents, taste masking agents, film modifiers, deodorants, gum soothing agents for irritation relief, and/or one or more therapeutic agents.

Suitably, an antimicrobial enhancing agent may be included and is selected from ethylenediamine tetraacetic acid, propionic acid, and cyclodextrin and its derivatives.

Suitably, a plasticizer may be included and is selected from triethyl citrate, polyethylene glycol, diethyl phthalate, dibutyl sebecate and triacetin.

Suitably, a flavorant may be included and is selected from an essential oil such as eucalyptus, cornmint oil, methyl salicylate, lemon, peppermint, tea tree common, tea tree, thyme, spearmint, menthol, and combinations thereof.

Suitably, a control release agent may be included and is selected from sodium bicarbonate, or a cross-linking agent such as lysine or polylysine, chitosan or chitin.

Suitably, a taste masking agent may be included and is selected from sucrolose, sodium saccharin, xylitol, aspartame, and other artificial sweeteners known in the art.

Suitably, a film modifier may be included and is selected from a clay additive such as montmorillonite, bentonite, and laponite; a detackifying agent such as talc, and aluminum hydrate; or an anti-caking agent such as silica.

Suitably, a deodorant and/or breath freshening agent may be included and is selected from cyclodextrin and its derivatives, or activated carbon.

Suitably, a gum soothing agent to improve gum health and to reduce gum irritation may be included. Some examples of such gum soothing agents include but are not limited to, aloe vera, tea tree oil, clove oil, sodium hyaluronate, chamomile, thyme oil, palmarosa oil, and epigallocatechin gallate.

Suitably, a therapeutic agent may be included and the denture care composition serves as a delivery vehicle for such therapeutic agent. Therapeutic actives that are useful in these compositions include antimicrobial agents such as iodine, sulfonamides, bisbiguanides, or phenolics; antibiotics such as tetracycline, neomycin, kanamycin, metronidazole, or clindamycin; anti-inflammatory agents such as aspirin, acetaminophen, naproxen and its salts, ibuprofen, ketorolac, flurbiprofen, indomethacin, cimetidine, eugenol, or hydrocortisone; anesthetic agents such as lidocaine or benzocaine; anti-fungals; aromatics such as camphor, eucalyptus oil, and aldehyde derivatives such as benzaldehyde; insulin; steroids; and anti-neoplastics. It is recognized that in certain forms of therapy, combinations of these agents in the same delivery system may be useful in order to obtain an optimal effect.

The denture care composition of this invention can be made by conventional methods of mixing the film-forming polymer with the solvent vehicle, heating the mixture and adding the desired excipients, such as a sweetener, cooling the mixture and then adding the flavor mix.

The denture care composition of this invention can be delivered in any suitable packaging options, including, but not limited to, a spray bottle with either an extended spray nozzle or flat spray nozzle; a dispensing pen having either a push button tip, a screw down tip, a marker tip, or a brush tip; a varnish bottle such as that used in commercial product Simply Whitening Night Gel package; a tube with two different tips at each end, one for the upper denture and one for the lower denture; a foam tip dispenser; a unit dose disposable swab; a unit dose disposable brush, a unit dose disposable pouch, swab, or marker tip; or a swab with a separate container having the denture care composition.

The composition may also be part of a kit having an appropriate dispenser for the denture care composition and any other suitable oral healthcare device, for example a toothbrush, toothpaste, a denture adhesive, a denture cleanser tablet/powder, a denture bath, and a denture cleaning wipe, as part of a total oral hygiene program for the denture wearer.

### EXAMPLES

### Example 1

The composition of the invention was prepared in a mixing vessel having heating/cooling capabilities and equipped with a variable speed mixer. With the mixer on, Eudragit L-100 was charged into the mixing vessel that contains propylene glycol. The jacketed vessel was heated to 45-50°C and mixed until visually uniform and translucent. When the temperature reached 45-50°C, heating was discontinued. In a separate container, sodium saccharin was dissolved in purified water. The sodium saccharin/water pre-mix was added to the main mixing vessel containing Eudragit L-100 and propylene glycol. The batch was then cooled down. In a separate container, flavor components were mixed until completely dissolved. When the batch mixture in the main mixing vessel cooled down to 20-25°C, the flavor pre-mix was added and mixed for approximately 10 minutes. Formulations included within the scope of this invention can be found in Table I below.

**TABLE 1**

| Formulations | Ex. A | Ex. B | Ex. C | Ex. D | Ex. E | Ex. F | Ex. G | Ex. H | Ex. I | Ex. J |
|---|---|---|---|---|---|---|---|---|---|---|
| Ingredient | Wt.% | Wt.% | Wt.% | wt.% | % | % | % | % | % | % |
| Propylene Glycol USP | 70.2 | 64.4 | 85.8 | 75.8 | 70.8 | 84.0 | 83.3 | 81.5 | 81.3 | 67.1 |
| Eudragit L100 | 2.5 | 5.0 | 7.5 | 7.5 | 7.5 | 10.0 | 10.0 | 12.5 | 12.5 | 15.0 |
| Purified Water | 25.0 | 30.0 | 5.0 | 15.0 | 20.0 | 5.0 | 5.0 | 5.0 | 5.0 | 15.0 |
| Flavor Mix | 2.0 | 0.5 | 1.5 | 1.5 | 1.5 | 0.8 | 1.5 | 0.8 | 1.0 | 2.5 |
| Na Saccharin USP | 0.3 | 0.1 | 0.2 | 0.2 | 0.2 | 0.2 | 0.2 | 0.2 | 0.2 | 0.4 |
| Total | 100.0% | 100.0% | 100.0% | 100.0% | 100.0% | 100.0% | 100.0% | 100.0% | 100.0% | 100.0% |

The above description fully discloses the invention including preferred embodiments thereof. Modifications and improvements of the embodiments specifically disclosed herein are within the scope of the following claims. Without further elaboration it is believed that one skilled in the art can, given the preceding description, utilize the present invention to its fullest extent. Therefore any examples are to be construed as merely illustrative and not a limitation on the scope of the present invention in any way. The embodiments of the invention in which an exclusive property or privilege is claimed are defined as follows.

## Claims

1. A liquid denture care composition, free of an antimicrobial agent and ethanol, comprising one or more sustained release polymers selected from a copolymer or terpolymer of methacrylic acid and methyl methacrylate; a polyvinyl acetate polymer; a copolymer of ethyl acrylate and methyl methacrylate; a copolymer of polyvinyl alcohol and polyethylene glycol; a copolymer of methacrylic acid and ethyl acrylate; a copolymer of vinylpyrrolidone and vinyl acetate; and a polyvinylpyrrolidone homopolymer, in a solvent vehicle which is propylene glycol present in the amount of between 50 and 90 weight percent of the total formulation.

2. The liquid denture care composition as claimed in claim 1, wherein the sustained release polymer is a copolymer or terpolymer of methacrylic acid and methyl methacrylate.

3. The liquid denture care composition as claimed in claim 2, wherein the copolymer or terpolymer of methacrylic acid and methyl methacrylate is a Eudragit® polymer.

4. The liquid denture care composition as claimed in claim 3, wherein the Eudragit® polymer is Eudragit® L-100.

5. A method for maintaining fresh breath in a denture wearer comprising applying the liquid denture care composition as claimed in claim 1 to the denture outside the mouth and inserting the denture into the denture wearer's mouth.

6. A method for the treatment and/or prophylaxis of denture odor in a denture wearer comprising applying the liquid denture care composition as claimed in claim 1 to the denture outside the mouth and inserting the denture into the denture wearer's mouth.

7. A kit for delivering a liquid denture care composition comprising a dispenser containing the liquid denture care composition as claimed in claim 1 and a suitable oral healthcare device as part of a total oral hygiene program for the denture wearer.

## Patentansprüche

1. Eine flüssige, Antimikrobiotikum- und Ethanol-freie flüssige Gebisspflegezusammensetzung, umfassend ein oder mehrere Polymere mit anhaltender Freisetzung, ausgewählt aus einem Copolymer oder Terpolymer von Methacrylsäure und Methylmethacrylat; einem Polyvinylacetatpolymer; einem Copolymer von Ethylacrylat und Methylmethacrylat; einem Copolymer von Polyvinylalkohol und Polyethylenglykol; einem Copolymer von Methacrylsäure und Ethylacrylat; einem Copolymer von Vinylpyrrolidon und Vinylacetat; und einem Polyvinylpyrrolidon-Homopolymer, in einem Lösungsmittelvehikel, das Propylenglykol ist, das in einer Menge von zwischen 50 und 90 Gew.-% der gesamten Formulierung vorliegt.

2. Die flüssige Gebisspflegezusammensetzung gemäß Anspruch 1, wobei das Polymer mit anhaltender Freisetzung ein Copolymer oder Terpolymer von Methacrylsäure und Methylmethacrylat ist.

3. Die flüssige Gebisspflegezusammensetzung gemäß Anspruch 2, wobei das Copolymer oder Terpolymer von Methacrylsäure und Methylmethacrylat ein Eudragit®-Polymer ist.

4. Die flüssige Gebisspflegezusammensetzung gemäß Anspruch 3, wobei das Eudragit®-Polymer Eudragit® L-100 ist.

5. Verfahren zum Erhalt eines frischen Atems eines Gebissträgers, umfassend Aufbringen der flüssigen Gebisspflegezusammensetzung gemäß Anspruch 1 auf das Gebiss außerhalb des Mundes und Einführen des Gebisses in den Mund des Gebissträgers.

6. Verfahren zur Behandlung und/oder Prophylaxe von Mundgeruch bei einem Gebissträger, umfassend Aufbringen der flüssigen Gebisspflegezusammensetzung gemäß Anspruch 1 auf das Gebiss außerhalb des Mundes und Einführen des Gebisses in den Mund des Gebissträgers.

7. Kit zur Abgabe einer flüssigen Gebisspflegezusammensetzung, umfassend einen Dispenser, der die flüssige Gebisspflegezusammensetzung gemäß Anspruch 1 enthält, und ein geeignetes orales Gesundheitspflegegerät als Teil eines gesamten oralen Hygieneprogramms für den Gebissträger.

## Revendications

1. Composition de soin de prothèse dentaire liquide, dépourvue d'agent antimicrobien et d'éthanol, comprenant un ou plusieurs polymères à libération prolongée choisis parmi un copolymère ou terpolymère d'acide méthacrylique et de méthacrylate de méthyle ; un polymère d'acétate de polyvinyle ; un copolymère d'acrylate d'éthyle et de méthacrylate de méthyle ; un copolymère d'alcool polyvinylique et de polyéthylène glycol ; un copolymère d'acide méthacrylique et d'acrylate d'éthyle ; un copolymère de vinylpyrrolidone et d'acétate de vinyle ; et un homopolymère de polyvinylpyrrolidone, dans un véhicule solvant qui est un propylène glycol présent en une quantité de 50 à 90 pour cent en poids de la formulation totale.

2. Composition de soin de prothèse dentaire liquide selon la revendication 1, dans laquelle le polymère à libération prolongée est un copolymère ou un terpolymère d'acide méthacrylique et de méthacrylate de méthyle.

3. Composition de soin de prothèse dentaire liquide selon la revendication 2, dans laquelle le copolymère ou terpolymère d'acide méthacrylique et de méthacrylate de méthyle est un polymère Eudragit®.

4. Composition de soin de prothèse dentaire liquide selon la revendication 3, dans laquelle le polymère Eudragit® est un Eudragit® L-100.

5. Procédé pour maintenir une haleine fraîche chez un porteur de prothèse dentaire, comprenant l'application de la composition de soin de prothèse dentaire liquide selon la revendication 1 à la prothèse dentaire à l'extérieur de la bouche et l'insertion de la prothèse dentaire dans la bouche du porteur de prothèse dentaire.

6. Procédé pour le traitement et/ou la prophylaxie de l'odeur d'une prothèse dentaire chez un porteur de prothèse dentaire, comprenant l'application de la composition de soin de prothèse dentaire liquide selon la revendication 1 à la prothèse dentaire à l'extérieur de la bouche et l'insertion de la prothèse dentaire dans la bouche du porteur de prothèse dentaire.

7. Kit de fourniture d'une composition de soin de prothèse dentaire liquide, comprenant un distributeur contenant la composition de soin de prothèse dentaire liquide selon la revendication 1 et un dispositif de soin buccal adapté, dans le cadre d'un programme d'hygiène buccale total pour le porteur de la prothèse dentaire.
